# EUROPÄISCHE PATENTANMELDUNG

(11) **EP 2 030 634 A1**
(43) Veröffentlichungstag der Anmeldung: **04.03.2009**
(21) Anmeldenummer: 08105034.6
(22) Anmeldetag: 13.08.2008
(51) Int. Cl.: A61K 49/22, A61K 47/48, A61K 9/00, A61P 35/00

(54) **Arzneimittel und Verfahren zur Behandlung von Prostatakrebs**

(30) Priorität: 03.09.2007 DE 102007041832
(71) Anmelder: Siemens Aktiengesellschaft, 80333 München (DE)
(72) Erfinder: Ergün, Süleyman, 45130, Essen (DE); Singer, Bernhard, 45147, Essen (DE); Tilki, Derya, 81377, München (DE); Fehre, Jens, 91353, Hausen (DE); Nanke, Ralf, 91077, Neunkirchen am Brand (DE); Stetter, Martin, 80997, München (DE)

(57) **Zusammenfassung**

Die Erfindung betrifft ein Arzneimittel zur Behandlung von Prostatakrebs und ein entsprechendes Behandlungsverfahren. Das Arzneimittel ist über die Blutbahn einem Prostatatumor (3) zuführbar und enthält eine Wirk- und eine Bindungskomponente sowie Ultraschall-Microbubbles (4), wobei
- die Bindungskomponente Koppelmoleküle (5) umfasst, die spezifisch an ein bestimmtes für den Tumor oder ein Tumorstadium typisches, im Endothel von tumorassoziierten Blutgefäßen (1) ausgebildetes Zielmolekül (10) binden,
- die Wirkkomponente wenigstens einen chemotherapeutischen Wirkstoff enthält, und
- Koppelmoleküle (5) an die Außenseite (7) der Ultraschall-Microbubbles (4) gebunden sind und Wirkstoffmoleküle (9) ebenfalls an die Außenseite (7) der Ultraschall-Microbubbles (4) gebunden oder von diesen umschlossen sind.

## Beschreibung

Die Erfindung betrifft ein Arzneimittel zur Behandlung von Prostatakrebs sowie ein entsprechendes Behandlungsverfahren. Über die Blutbahn einem Prostatatumor zuführbare Arzneimittel werden mangels geeigneter Diagnoseverfahren für Vorstadien von Prostatatumoren erst dann verabreicht, wenn der Tumor schon fortgeschritten ist, sich beispielsweise schon im Stadium der Angiogenese befindet. Dementsprechend gering sind dann oftmals die Heilungschancen.

Davon ausgehend ist es die Aufgabe der Erfindung, ein Arzneimittel und ein Behandlungsverfahren vorzuschlagen, mit welchen die Heilungschancen verbessert werden.

Diese Aufgabe wird durch ein Arzneimittel nach Anspruch 1 und ein Behandlungsverfahren nach Anspruch 11 gelöst. Ein erfindungsgemäßes Arzneimittel enthält eine Wirk- und eine Bindungskomponente sowie Microbubbles. Die Bindungskomponente umfasst Koppelmoleküle, die spezifisch an CEACAM-1 binden. Die Wirkkomponente enthält wenigstens einen chemotherapeutischen Wirkstoff. Die Microbubbles fungieren dabei als Träger für Zielmoleküle und Wirkstoffmoleküle, wobei erstere an die Außenseite der Ultraschall-Microbubbles gebunden sind. Auch für die Wirkstoffmoleküle bietet sich diese Möglichkeit an. Diese können aber auch innerhalb der Ultraschall-Microbubbles vorhanden, also von diesen umschlossen sein, wobei sie dann vor jeglicher Beeinträchtigung während des Transports zum Zielort verschont sind. Ein solches Arzneimittel verteilt sich beispielsweise nach intravenöser Verabreichung im Körper. Aufgrund der an der Oberfläche der Microbubbles vorhandenen Koppelmoleküle kommt es aber zu einer gezielten Bindung und Anreicherung in einem Gewebe, in dem CEACAM-1 vorhanden ist. Die Erfindung stützt sich dabei auf Forschungsergebnisse von Tilki et al. (Oncogene (2006)25,4965-4974), gemäß denen CEACAM-1 bei einer gesunden Prostata nur in deren Epithel, nicht jedoch in den Blutgefäßen der Prostata vorkommt. In einer frühen Phase der Tumorentwicklung, etwa im Stadium der hgPIN (high grade epithelial prostatic neoplasia), in welcher der Tumor noch keine eigenen Gefäße ausbildet, ist die Expression von CEACAM-1 im Prostataepithel herunterreguliert, während sie im Endothel angrenzender kleiner Blutgefäße des gesunden Gewebes hochreguliert ist. Dieser Effekt wird nun erfindungsgemäß zur Erkennung eines Krebsfrühstadiums ausgenutzt. Im Gegensatz zu einer gesunden Prostata ergibt sich bei Vorhandensein eines Tumors im Frühstadium ein Anreicherungsbild. Hier erfolgt eine Konzentration des Kontrastmittels im Bereich des Tumors, so dass dieser im Sinne eines theranostischen Ansatzes zum einen beobachtet bzw. diagnostiziert und andererseits zielgerichtet therapeutisch durch extern induzierte Wirkstoff-Freisetzung behandelt werden kann.

Die Wirkstoff-Freisetzung innerhalb des Tumorgewebes wird dadurch ermöglicht, dass durch Ultraschall zerstörbare Microbubbles, also äußerst kleine, beispielsweise von einer Lipid-Doppelmembran gebildete Bläschen als Träger für die Wirkkomponente eingesetzt werden. Durch Beaufschlagung des Tumors mit Ultraschall vorzugsweise über eine Rektalsonde kann der Zeitpunkt der Wirkstoff-Freisetzung vom behandelnden Arzt frei gewählt werden. Aufgrund der Anreicherung wird dabei im Prostatatumor eine hohe therapeutisch wirksame Wirkstoffkonzentration erreicht, ohne dass Zellen gesunden Gewebes davon in nennenswertem Ausmaß betroffen sind. Bei der durch Ultraschall gesteuerten Freisetzung wird nämlich allenfalls nur ein Teil der Wirkstoffmoleküle vom Blutstrom mitgenommen, wobei sich aber deren Konzentration aufgrund der Verteilung im Kreislaufsystem stark verringert. Vorteilhaft ist weiterhin, dass das Arzneimittel gleichzeitig als Diagnostikum wirkt, da es den Prostatatumor erkennt. Bei Verwendung der entsprechenden Bindungskomponente lassen sich frühe Krebsstadien aufdecken und gezielt wirksam behandeln. Das Arzneimittel ist somit theranostisch einsetzbar, d.h. es dient sowohl diagnostischen als auch therapeutischen Zwecken. Da die Microbubbles auch als Kontrastmittel für ultraschallbasierte Bildgebungsverfahren wirken, kann die Anreicherung des Arzneimittels in der Prostata verfolgt und der Zeitpunkt der Wirkstoff-Freisetzung durch eine Ultraschallbeaufschlagung optimal gewählt werden. Außerdem ist es möglich, bei einer Behandlung den Erfolg vorangegangener Behandlungen mit Hilfe eines ultraschallbasierten Bildgebungsverfahren zu kontrollieren.

Für ein erfindungemäßes Verfahren zur Behandlung von Prostatakrebs, bei dem nach Verabreichung des Arzneimittels und dessen Anreicherung im Prostatatumor eine durch Ultraschall induzierte Wirkstoff-Freisetzung erfolgt, gelten die obigen Ausführungen analog.

In den Unteransprüchen sind vorteilhafte Weiterbildungen angegeben, die bei der folgenden, auf die beigefügten Zeichnungen Bezug nehmenden Beschreibung der Erfindung berücksichtigt werden. Es zeigen::
Fig. 1 eine in schematisierter Darstellung eines ein Blutgefäß und einen Tumor aufweisenden Bereichs der Prostata, welche die Anreicherung eines Arzneimittels im Tumor verdeutlicht,
Fig.2 eine Fig. 1 entsprechende Darstellung, die das Prinzip der gezielten Wirkstoff-Freisetzung innerhalb er Prostata zeigt.

Ein über ein Blutgefäß 1 der Prostata 2 und insbesondere eines dort vorhandenen Tumors 3 zugeführtes Arzneimittel enthält eine Bindungskomponente, eine Wirkstoffkomponente und als Träger für die genannten Komponenten fungierende Microbubbles 4. Die Bindungskomponente bewirkt eine gezielte Anreicherung des Arzneimittels. Sie umfasst Koppelmoleküle 5, die an CEACAM-1 (carzinoembryonic antigen-related cell adhesion molecule) als Zielmolekül 10 binden. Diese Moleküle sind, wie weiter oben bereits ausgeführt wurde, im Endothel der dem sich in einem Frühstadium befindlichen Tumor benachbarten Blutgefäße vorhanden. Die Koppelmoleküle sind an die Außenseite 7 der Microbubbles gebunden und zwar mittel- oder unmittelbar. Microbubbles 4 sind an sich bekannt. Sie umfassen eine beispielsweise aus einer Lipid-Doppelmembran gebildete Hülle, welche einen Hohlraum 8 umschließt.

Die Wirkkomponente umfasst Wirkstoffmoleküle 9, die entweder, wie die Koppelmoleküle 5, an die Außenseite der Microbubbles gebunden oder in deren Hohlraum 8 eingeschlossen sind. Letztere Variante garantiert, dass während des Transport der Microbubbles im Blutstrom jegliche Wechselwirkung mit Bestandteilen des Bluts ausgeschlossen ist. Außerdem steht auf der Außenseite 7 der Microbubbles 4 eine größere Fläche zur Aufnahme von Koppelmolekülen zur Verfügung. Als Wirkstoffe kommen praktisch alle zur Bekämpfung des Prostatatumors geeigneten Therapeutika wie Docetaxel und beispielsweise auch Wirkstoffe wie Angiogenese-Hemmer in Frage, welche die Neubildung von Blutgefäßen verlangsamen oder verhindern. Wegen der Kapselung der Wirkstoffe in den Microbubbles können auch Giftstoffe eingesetzt werden, die bei herkömmlicher Anwendung den Körper schädigen würden. Die Microbubbles können jeweils nur mit einem einzigen Wirkstoff oder mehreren unterschiedlich therapeutisch wirksamen Wirkstoffe bzw. Wirkstoffmolekülen beladen sein. Wie bereits erwähnt, erfüllen die Microbubbles erfindungsgemäß eine Doppelfunktion, indem sie einerseits als Träger für die Wirkstoffkomponente und andererseits als Kontrastmittel für die Bildgebung dienen. Die Kontrastverstärkung kann bei mit Wirkstoffmolekülen beladenen, insbesondere gefüllten Microbubbles 4 abgeschwächt sein, so dass es zweckmäßig ist, wenn das Arzneimittel wirkstofffreie Microbubbles 4 enthält, die sich hinsichtlich ihrer Bindungswirkung d.h. der an ihrer Außenseite vorhandenen Koppelmoleküle 5, nicht von den Wirkstoffmoleküle tragenden Microbubbles 4 unterscheiden.

Die zielgerichtete Anreicherung in Frühstadien des Prostatatumors 3 wird über die jeweiligen Bindungspartner, d.h. Koppelmoleküle 5 einerseits und gewebetypische Zielmoleküle 10, in erster Linie also CEACAM-1, andererseits bewerkstelligt.

Im Vorstadium des Prostatatumors, beispielsweise dem Stadium der hochgradigen intraepithelialen prostatischen Neoplasie (hgPIN) hat dieser noch keine Blutgefäße 1 entwickelt. Als Folge des Tumors bilden sich aber im Endothel benachbarter Blutgefäße CEACAM-1-Moleküle aus. Ein Arzneimittel, das an CEACAM-1 spezifisch bindende Koppelmoleküle, insbesondere CEACAM-1-Antikörper enthält, kann somit z.B. hgPIN erkennen und sich gezielt in dem entsprechenden Gewebe anreichern, so dass dieses Stadium mit Hilfe einer Ultraschall-Bildgebung sichtbar gemacht wird. Weiter fortgeschrittene Karzinome regen die Angiogenese, die Bildung von Blutgefäßen 1 an. Die Angiogenese begleitende Wachstumsfaktoren wie beispielsweise VEGF (vasco endothelial growth factor) dienen dabei als Zielmoleküle 10, mit denen entsprechende Koppelmoleküle 5 als Bindungspartner, beispielsweise VEGF-Liganden oder - Antikörper, zusammenwirken. In den bisher genannten Fällen können als Koppelmoleküle 5 auch Aptamere, Spiegelmere oder Anticaline dienen. Aptamere sind kurze, stabile und spezifisch bindende RNA-Ketten, Spiegelmere sind die spiegelbildlichen Pendants der Aptamere. Bei den Anticalinen handelt es sich um einzelne Polypeptidketten mit ca. 180 Aminosäuren, die ähnlich spezifische Bindungseigenschaften aufweisen wie Antikörper, die aber leichter herstellbar sind diese.

Auch in einem späteren Krebsstadium kann es zweckmäßig sein, ein Arzneimittel zu verwenden, das einen Anteil bzw. eine Charge von Microbubbles enthält, deren Außenseite 7 mit Koppelmolekülen 5 versehen ist, die an CEACAM-1 binden. Beispielsweise hilft eine solche CEACAM-1 vermittelte Markierung, den Anteil angiogenetisch aktiver Blutgefäße am Tumorgefäßbett zu bestimmen bzw. das Reagieren der Tumorgefäße auf eine anti-angiogenetische Therapie in der Bildgebung erfassen zu können. Denkbar ist auch, dass die Verabreichung eines derartigen Arzneimittels mit einem Arzneimittel kombiniert wird, das Microbubbles 4 mit an VEGF bindenden Koppelmolekülen enthält, um innerhalb des Tumorgefäßbettes eine gewisse Differenzierung der Gefäße hinsichtlich ihrer Aktivität vornehmen zu können. Es kann auch ein Arzneimittel verabreicht werden, das beide der genannten Arten von Microbubbles enthält, so dass komplexe Krebsstadien erkannt werden.

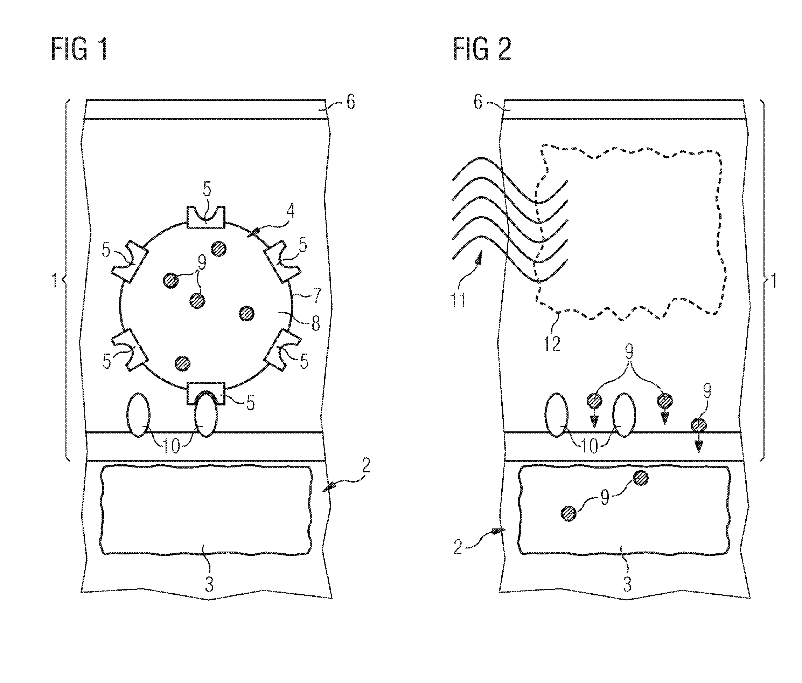

Eine Ultraschall-Theranostiksitzung kann beispielsweise wie folgt ablaufen: Dem Patienten wird eine Charge molekular markierter, also spezifisch bindende Koppelmoleküle 5 aufweisender, mit Wirkstoff beladener Microbubbels in dem Blutstrom injiziert. Hat der Patient Prostatakrebs im frühen oder mittleren Stadium, so reichern sich die Microbubbles selektiv im Tumor 3 bzw. in dessen Umfeld an, indem deren Koppelmoleküle an endotheliale CEACAM-1-Moleküle binden. Simultan dazu wird mit Hilfe eines Ultraschallgerätes bzw. einer Rektalsonde eine Ultraschallaufnahme der Prostata gemacht. Dabei wirken die Microbubbles kontrastverstärkend. Der Anreicherungsprozess der Microbubbles kann somit verfolgt werden. Da die Anreicherung nur im Tumor erfolgt, kann dessen Vorhandensein und Ausdehnung diagnostiziert werden. Sind der gegenwärtigen Sitzung schon weitere Theranostiksitzungen vorausgegangen, so stellt dieser Schritt gleichzeitig eine Verlaufskontrolle dar: Durch Vergleich mit den vorangegangenen Tumorbildern kann festgestellt werden, ob und in welchem Ausmaß der Tumor sich zurückgebildet hat. Ist ein Tumor 3 vorhanden und ist der Anreicherungsprozess genügend weit fortgeschritten, so löst der Arzt einen Ultraschallpuls 11 aus. Dieser ist so beschaffen, dass er die Microbubbels zerstört (angedeutet durch die gestrichelte Linie 12 in Fig. 2). Dadurch werden die Wirkstoffmoleküle 9 zielgenau und nicht invasiv im Tumorgewebe freigesetzt und können dort ihre Wirkung mit maximaler Schlagkraft entfalten. Nicht gebundene Microbubbles befinden sich an anderen Stellen im Körper und werden daher durch den Ultraschallpuls 11 nicht zerstört, so dass gesundes Gewebe von der Einwirkung der Wirkstoffmoleküle 5 verschont bleibt.

## Patentansprüche

1. Arzneimittel zur Behandlung von Prostatakrebs, welches über die Blutbahn einem Prostatatumor (3) zuführbar ist, enthaltend eine Wirk- und eine Bindungskomponente sowie Ultraschall-Microbubbles (4), wobei
- die Bindungskomponente von Koppelmolekülen (5) gebildet ist, die spezifisch an CEACAM-1 binden,
- die Wirkkomponente wenigstens einen chemotherapeutischen Wirkstoff enthält, und
- Koppelmoleküle (5) an die Außenseite (7) der Microbubbles (4) gebunden sind und Wirkstoffmoleküle (9) ebenfalls an die Außenseite (7) der Microbubbles (4) gebunden oder von diesen umschlossen sind.

2. Arzneimittel nach Anspruch 1, das Microbubbles (4) enthält, die nicht mit Wirkstoffmolekülen (9) beladen sind.

3. Arzneimittel nach Anspruch 1 oder 2, das Microbubbles (4) enthält, die mit unterschiedlichen Wirkstoffmolekülen (9) beladen sind.

4. Arzneimittel nach Anspruch 3, das mit einem antiangiogenetischen Wirkstoff und einem chemotherapeutischen Wirkstoff beladene Microbubbles (4) enthält.

5. Arzneimittel nach einem der vorhergehenden Ansprüche, das CEACAM-1-Antikörper als Koppelmoleküle (5) enthält.

6. Arzneimittel nach Anspruch 5, das als Koppelmoleküle (5) Aptamere, Spiegelmere und/oder Anticaline enthält.

7. Arzneimittel nach einem der vorhergehenden Ansprüche, mit Koppelmolekülen (5) , die an für ein späteres, auf das hgPIN-Stadium folgendes Tumorstadium typisch sind.

8. Arzneimittel nach Anspruch 7, mit an den Blutgefäß-Wachstumsfaktor VEGF bindenden Koppelmolekülen (5).

9. Arzneimittel nach Anspruch 8, mit VEGF-Liganden oder - Antikörpern als Koppelmoleküle (5).

10. Arzneimittel nach Anspruch 8, das als Koppelmoleküle (5) Aptamere, Spiegelmere und/oder Anticaline enthält.

11. Verfahren zur Behandlung von Prostatakrebs mit einem Arzneimittel nach einem der vorhergehenden Ansprüche, bei dem einem Prostatatumor (3) über die Blutbahn das Arzneimittel zugeführt und anschließend die Microbubbles (4) mit Hilfe eines Ultraschallpulses (11) zerstört werden.

12. Verfahren nach Anspruch 11, bei dem vor der Zerstörung der Microbubbles (4) eine Ultraschallaufnahme des Prostatatumors (3) durchgeführt wird.
